# EUROPEAN PATENT SPECIFICATION

(11) **EP 2 569 570 B1**
(45) Date of publication and mention of the grant of the patent: **15.08.2018**
(21) Application number: 11780996.2
(22) Date of filing: 20.04.2011
(51) Int. Cl.: F17C 9/00, A61L 2/02, A61L 2/04

(54) **METHOD FOR PRODUCING STERILE CRYOGENIC LIQUID**
VERFAHREN ZUR HERSTELLUNG EINER STERILEN KRYOGENEN FLÜSSIGKEIT
PROCÉDÉ DE PRODUCTION DE LIQUIDE CRYOGÉNIQUE STÉRILE

(30) Priority: 12.05.2010 US 778299
(43) Date of publication of application: 20.03.2013
(73) Proprietor: Linde AG, 80331 Munich (DE)
(72) Inventor: LEE, Ron, C., Bloomsbury, NJ 08804 (US)
(74) Representative: Gellner, Bernd
(86) International application number: PCT/US2011/033202
(87) International publication number: WO 2011/142945

(56) References cited:
- EP-A1- 1 707 866
- EP-A2- 0 092 795
- US-A- 4 620 962
- US-A- 4 865 088
- US-A- 4 865 088
- US-A- 5 557 924
- US-A- 5 557 924
- US-A- 5 749 232
- US-A1- 2003 029 179
- US-A1- 2005 011 201

## Description

### BACKGROUND OF THE INVENTION

The present invention relates to a method and apparatus for the production of a sterile cryogenic fluid such as liquid nitrogen. The invention employs an improved pressure and flow control arrangement which significantly reduces the complexity of the system and the validation requirements for steam sterilization.

Earlier methods for the production of sterile cryogenic fluids such as liquid nitrogen have generally used two broad approaches. The first method uses direct liquid filtration (down to about 0.2 micron or less) to remove microbial or other contaminants. The second method also uses filtration to about 0.2 micron or less, but starts by filtering warm gas. This sterile warm gas is then liquefied in an indirect heat exchange process with a suitable low temperature refrigerant. This low temperature refrigerant is generally liquid having the same chemical composition as the sterile gas (e.g., nitrogen). In this case it is necessary to maintain the sterile warm gas, and the sterile liquid while being produced, at a pressure significantly higher than that of the liquid refrigerant. This generally introduces piping and valve complexities, and makes it difficult to pre-sterilize the system (typically using steam). The second method is represented in US 4,620,962 and EP1707866A1.

Numerous patents exist for liquid nitrogen sterilization according to the two methods of direct liquid sterilization, or gas sterilization and subsequent liquefaction. Typical patents associated with the latter approach are US 4,620,962 and EP 0888132B1. US'962 shows an arrangement very similar to an embodiment of the present invention that includes a coil 11 which could conceivably introduce a pressure drop similar to that envisioned in the present invention. However, US'962 references US 4,510,760 which clearly shows coil 11 is associated with supplemental heat exchange with no mention or envisioning of controlled pressure drop according to the present invention.

US 5,557,924 teaches methods for the metered delivery through closing of an on/off valve of a purified liquid cryogen. This is accomplished by positioning a cryogenic sterilizer in the delivery conduit such that it will purify the cryogen flowing there through. The cryogen purifier and the clean delivery conduit section are isolated from the source of the liquid cryogen during purification and this purification will occur without subject the liquid cryogen source to the cleansing medium.

US 4,865,088 teaches means to control a stream of liquid cryogen by sub-cooling the cryogen to keep the flow primarily liquid. The rate at which liquid cryogen is delivered at the outlet is controlled by the cross-sectional area of the flow control restriction.

EP 0 092 795 A2 recognizes that at constant pressure and fixed liquid level, the outflow rate is controlled by the nozzle hole diameter. This invention improves on this design through the use of an outflow nozzle having a plurality of through-holes running through the base of the heat-insulating container for controlling the flow of the low temperature liquid.

US 2003/0029179 A1 teaches methods for controlling the temperature of a system to provide for flow control of cryogen from a storage tank to an evaporator coil. By measuring these temperatures at two locations, consistent flow can be maintained in response to the temperature changes.

### SUMMARY OF INVENTION

According to the present invention, there is disclosed a method according to claim 1. Advantageous embodiments and expedient further developments of the present invention are disclosed in the dependent claims.

An important property that the discharge orifice should possess is its ability to be steam sterilized. Unlike a needle valve, the orifice should have no traps or pockets like a needle valve and can be readily sterilized with steam. The orifice should provide a reproducible and constant flow restriction that will offer a controlled pressure drop along the line leading to the orifice.

### BRIEF DESCRIPTION OF DRAWINGS

Fig. 1 is schematic showing a tube in tube arrangement for the production of a sterile cryogenic fluid.
Fig. 2 is a schematic of a simple coiled tube in a liquid nitrogen bath arrangement for the production of sterile liquid nitrogen.
Fig. 3 shows two alternative coiling arrangements.

### DETAILED DESCRIPTION OF THE INVENTION

The present invention provides for optimizing the discharge rate of a sterile cryogenic fluid from a sterile cryogenic fluid production process by determining the optimum discharge orifice size based upon a predetermined flow rate and pressure of the sterile cryogenic fluid.

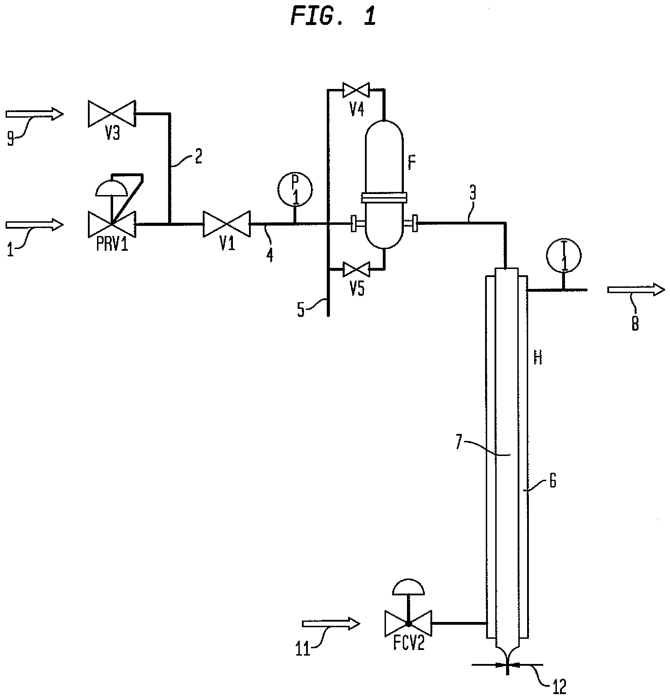

The method of the present invention is illustrated using a specific example. Fig. 1 provides a simple arrangement where the object is to produce a flow of sterile liquid nitrogen for delivery at atmospheric pressure. Normal gaseous nitrogen 1 at a predetermined pressure is fed through valve PRV1 to a standard sterilizing filter to produce a sterile nitrogen gas stream 3. This sterile nitrogen gas stream is then fed through a simple tube in tube heat exchanger H where it is liquefied 7 through indirect heat exchange with a liquid nitrogen cooling stream 6 which is at close to atmospheric pressure. The key element of the present invention is the smooth orifice 12 that is placed at the discharge of the sterile liquid nitrogen. The discharge orifice is sized to deliver liquid nitrogen at a predetermined flow at the predetermined pressure.

The predetermined pressure must be sufficiently higher than the pressure of liquid nitrogen cooling stream 6 to ensure the sterile gas stream will liquefy against the liquid nitrogen in the outer tube. For example, with a discharge to atmospheric pressure the liquid nitrogen in the outer tube will be at approximately -196°C. It is necessary for the sterile gas to liquefy at a temperature at least 5 to 10 degrees warmer than that (about -191 °C to - 186°C), which is about 0.7 to 1.8 barg in this example. The pressure of the liquid nitrogen cooling stream 6 may be above or below atmospheric pressure. That will affect the minimum predetermined pressure in order to satisfy the requirement of 5 to 10°C difference in saturation temperatures. For this example, selecting a predetermined pressure of 3.3 barg and a predetermined flow of about 54 kg/hr, the required orifice size is approximately 1 mm. In practice, the exact flow can be adjusted by a combination of reasonable adjustment of the operating pressure and/or orifice size. A final requirement for operation is that the surface area of the tube in tube (or equivalent heat exchange arrangement) is sufficient to accomplish the necessary heat exchange. Methods for sizing the heat exchanger, both analytical and experimental, are well known to those skilled in the art.

The sterile liquid nitrogen flow in the above example is self limiting and stable because if in fact a portion of the sterile gas were to be uncondensed at the discharge orifice the flow would dramatically reduce. In the extreme case of pure cold sterile gas on discharge, the fixed orifice and operating pressure of the above example would restrict the flow to only about 1.9 Kg/min because of the much lower density of gaseous nitrogen. That is less than 4% of the expected liquid flow capacity. Therefore if the amount of heat exchanger surface area is insufficient to effect complete condensation of the sterile gas flow, the system will self-stabilize to a lower flow because of the flow restriction caused by the presence of sterile gas at the discharge.

Flow control of the outer liquid nitrogen 11 in Fig. 1 for the above example could be through monitoring the vent nitrogen temperature and maintaining a temperature suitably warmer than the saturation temperature of liquid nitrogen. Suitable setpoint temperatures would be in the range of about -150°C to -50°C which would be maintained by adjusted FCV2 in Fig. 1. The colder setpoint temperature would improve heat transfer rates, while the warmer temperature would improve thermodynamic efficiency.

A significant advantage of the present invention is that the piping downstream of the sterile filter is unencumbered with any unnecessary fittings, valves, temperature or pressure sensors that will make sterile operation, and associated steam sterilization difficult. A simple arrangement for steam sterilization is shown in Fig. 1. Additional features, such as auxiliary heating, purge nitrogen, etc. are possible but not shown. For example, additional heat may be applied near the region of the discharge orifice in order to maintain the condensate at the minimum temperature necessary for adequate pre-sterilization. With the system warm, steam 9 is introduced into the system 2 *through open valve V1* by opening valves V3, V4 and V5 (the latter two valves are normal valves associated with steam sterilization of the filter). *Open valve V1 is then closed.*

Steam will naturally flow through the remainder of the system and out of the discharge orifice 12. The only useful precaution in the piping arrangement downstream of the sterile filter is to minimize, and preferably eliminate, piping traps and dead zones, and ensure adequate downward sloping of all lines to ensure the condensate will collect at the discharge orifice. The discharge orifice in this case will act in a manner very similar to a steam trap because liquid condensate will generally flow freely through the orifice 12, while uncondensed steam is severely restricted. For example, using 2.3 barg steam and the same 1 mm discharge orifice of the example, as much as 58 kg/hr of liquid condensate can be discharged with a system heating rate of over 34 kW. However, the amount of heating provided by the steam will be limited by the size and temperature of the system. This means less than the maximum amount of steam heating (34 kW in this case) will generally be required and therefore less than the maximum amount of steam condensate will form. In that case, some uncondensed steam will periodically be delivered to the discharge orifice.

The discharge orifice 12 will only permit a flow of about 2 Kg/hr of uncondensed steam, which is less than 4% of the expected liquid flow capacity. Therefore, once again the system is self limiting and stable relative to gas/liquid flow out of the discharge orifice 12. Many alternative arrangements for the heat exchange system shown in Fig. 1 are possible within the scope of the present invention.

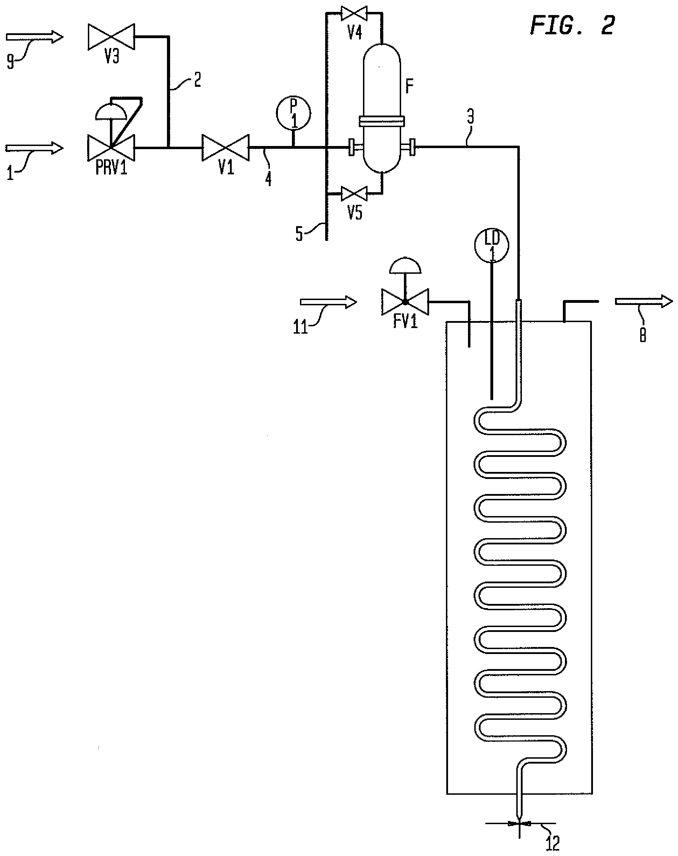

Fig. 2 shows a bath of liquid nitrogen with coils of tubing containing the sterile gas to be condensed. The same numbering scheme is employed in Fig. 2 as in Fig. 1 with the tube in tube arrangement being replaced by a simple coiled tube arrangement. Here the refrigerant liquid nitrogen flow 11 is shown controlled by a fill valve FV1 activated using a level detector LD1.

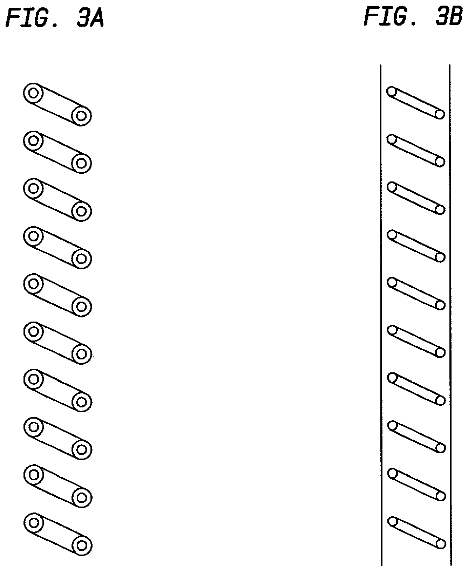

Fig. 3 shows alternative coiling arrangements that are possible for the tubing. Fig. 3(a) shows a tube in tube coil and Fig. 3(b) shows a coiled tube in outer pipe.

Any number of cryogenic fluids in addition to liquid nitrogen is possible. For the purposes of this invention, we may assume a cryogenic fluid is any liquid with a normal boiling point below -50°C, for example, oxygen, liquid air, and argon.

The method of warm sterilization is normally by filtration (typically with pore size of about 0.2 micron or less), but may also be any combination of filtration and/or other treatment technologies.

The cryogenic and ambient temperature fluid sources may be from single or multiple storage tanks or processes. For example, a single pressurized cryogenic liquid storage tank may be used to supply cryogenic liquid and warm vapor to the sterilization modules at various pressures and flow rates. Not shown are the various pressure regulation devices and flow control valves that would be known to those skilled in the art.

Additional heaters or ambient vaporizers may be added to perform additional warming or vaporization of the cryogenic fluids. Additional components may be added to further facilitate steam sterilization or similar processes in order to prepare the system for operation. This may include auxiliary heating elements to facilitate achieving minimum pre-sterilization temperatures in certain locations.

Downstream piping from the discharge orifice to a remote application point is possible, preferably with continued downward sloping. Reasonable pressure drop in the downstream piping may be accommodated within the scope of the invention by suitable adjustment of the setpoint pressure of the supplied gas to be sterilized.

The liquid cryogen used to liquefy the sterile gas may be vented at pressures greater than atmospheric, possibly including the addition of a back pressure regulator. This will necessitate a corresponding adjustment to the setpoint pressure of the gas introduction.

Additional heat exchangers may be added to further improve the thermodynamic efficiency of the process. For example, the cold vent gas that would be produced in the apparatus shown in Fig. 2 may be routed to an upstream heat exchanger that pre-cools the sterile gas prior to liquefaction, or the coil arrangements shown in Fig. 2 and 3 may further use the boil-off vapor in a manner similar to Fig. 1. Alternatively, the warm nitrogen gas may be produced by vaporization of liquid nitrogen. That vaporization may be at least partially through indirect heat exchange with the sterile warm gas and therefore reduce the amount of liquid required in the liquefaction chamber.

Flow control of the amount of sterile liquid produced is possible, which could include replacing the pressure regulating valve PRV1 with a suitable throttling control valve. The only limits are that sterile gas pressure must be sufficient to allow liquefaction, and the flow must be less than the maximum that can be condensed with the available heat exchange surface area.

Steam (thermal) sterilization as described herein is the normal, and generally preferred, method for sterilization of the equipment. However, alternative methods such as hydrogen peroxide or ozone sterilization methods are possible within the scope and intent of the present invention.

While this invention has been described with respect to particular embodiments thereof, it is apparent that numerous other forms and modifications of the invention will be obvious to those skilled in the art. The appending claims in this invention generally should be construed to cover all such obvious forms and modifications which are within the scope of the present invention.

## Claims

1. A method for producing a sterile cryogenic liquid by sterilizing a cryogenic gas (1) to produce a sterile cryogenic gas stream (3) and feeding the sterile cryogenic gas stream (3) to a heat exchanger (H) to liquefy (7) the sterile cryogenic gas stream (3),
**characterized by**
controlling a discharge rate of the sterile cryogenic liquid by sizing a discharge orifice (12) thereby to provide flow restriction of the sterile cryogenic liquid and by setting a predetermined pressure of the sterile cryogenic liquid wherein said predetermined pressure is higher than atmospheric pressure.

2. The method as claimed in claim 1 wherein said cryogenic liquid is selected from the group of liquids having a normal boiling point below -50°C.

3. The method as claimed in claim 2 wherein said cryogenic liquid is selected from the group consisting of nitrogen, oxygen, liquid air and argon.

4. The method as claimed in claim 1 wherein said sterilizing is warm sterilization by filtration.

5. The method as claimed in claim 1 further comprising steam sterilization of said heat exchanger (H) and discharge orifice (12).

6. The method as claimed in claim 1 wherein the pressure of said cryogenic gas is sufficient to allow liquefaction in said heat exchanger (H).

7. The method as claimed in claim 1 wherein said heat exchanger (H) has sufficient surface area to condense said cryogenic gas.

## Patentansprüche

1. Verfahren zur Herstellung einer sterilen kryogenen Flüssigkeit durch Sterilisieren eines kryogenen Gases (1) zur Herstellung eines sterilen kryogenen Gasstroms (3) und Zuführen des sterilen kryogenen Gasstroms (3) zu einem Wärmetauscher (H) zum Verflüssigen (7) des sterilen kryogenen Gasstroms (3),
**dadurch gekennzeichnet, dass**
eine Austrags rate der sterilen kryogenen Flüssigkeit durch Bemessung einer Austrittsöffnung (12), wodurch eine Strömungsbeschränkung der sterilen kryogenen Flüssigkeit bereitgestellt wird, und durch Einstellen eines vorbestimmten Drucks der sterilen kryogenen Flüssigkeit, wobei der vorbestimmte Druck höher als Normaldruck ist, gesteuert wird.

2. Verfahren nach Anspruch 1, bei dem die kryogene Flüssigkeit aus der Gruppe von Flüssigkeiten mit einem Normalsiedepunkt unter -50 °C ausgewählt wird.

3. Verfahren nach Anspruch 2, bei dem die kryogene Flüssigkeit aus der Gruppe bestehend aus Stickstoff, Sauerstoff, flüssiger Luft und Argon ausgewählt wird.

4. Verfahren nach Anspruch 1, bei dem es sich bei der Sterilisation um eine Warmsterilisation durch Filtration handelt.

5. Verfahren nach Anspruch 1, ferner umfassend eine Dampfsterilisation des Wärmetauschers (H) und der Austragsöffnung (12).

6. Verfahren nach Anspruch 1, bei dem der Druck des kryogenen Gases dazu ausreicht, eine Verflüssigung in dem Wärmetauscher (H) zu erlauben.

7. Verfahren nach Anspruch 1, bei dem der Wärmetauscher (H) eine zur Kondensation des kryogenen Gases ausreichende Oberfläche aufweist.

## Revendications

1. Procédé de production d'un liquide cryogénique stérile par stérilisation d'un gaz cryogénique (1) afin de produire un courant gazeux cryogénique stérile (3) et introduction du courant gazeux cryogénique stérile (3) dans un échangeur thermique (H) afin de liquéfier (7) le courant gazeux cryogénique stérile (3),
**caractérisé par**
la régulation d'un débit de sortie du liquide cryogénique stérile par réglage de la taille d'un orifice de sortie (12) de façon à assurer ainsi une restriction d'écoulement du liquide cryogénique stérile et établissement d'une pression prédéterminée du liquide cryogénique stérile, ladite pression prédéterminée étant supérieure à la pression atmosphérique.

2. Procédé selon la revendication 1, dans lequel ledit liquide cryogénique est sélectionné dans le groupe des liquides présentant un point d'ébullition normal inférieur à -50 °C.

3. Procédé selon la revendication 2, dans lequel ledit liquide cryogénique est sélectionné dans le groupe constitué de l'azote, de l'oxygène, de l'air liquide et de l'argon.

4. Procédé selon la revendication 1, dans lequel ladite stérilisation est une stérilisation à chaud par filtration.

5. Procédé selon la revendication 1, comprenant en outre une stérilisation à la vapeur dudit échangeur thermique (H) et dudit orifice de sortie (12).

6. Procédé selon la revendication 1, dans lequel la pression dudit gaz cryogénique est suffisante pour permettre une liquéfaction dans ledit échangeur thermique (H).

7. Procédé selon la revendication 1, dans lequel ledit échangeur thermique (H) présente une surface suffisante pour condenser ledit gaz cryogénique.
